(19) Europäisches Patentamt
European Patent Office
Office européen des brevets

(11) Veröffentlichungsnummer: **0 009 708**
**B1**

# (12) EUROPÄISCHE PATENTSCHRIFT

(45) Veröffentlichungstag der Patentschrift: **21.11.84**

(51) Int. Cl.³: **C 07 C 69/743,** A 01 N 53/00, C 07 F 9/40

(21) Anmeldenummer: **79103487.9**

(22) Anmeldetag: **17.09.79**

(54) **Substituierte Bromostyryl-cyclopropancarbonsäure-m-phenoxybenzylester (I),Verfahren zu ihrer Herstellung, hierbei erhaltene Zwischenprodukte und deren Herstellung, die Ester (I) enthaltende Mittel, Verwendung der Ester (I) und Verfahren zur Herstellung insektizider und/oder akarizider Mittel.**

(30) Priorität: **29.09.78 DE 2842542**

(43) Veröffentlichungstag der Anmeldung: **16.04.80 Patentblatt 80/8**

(45) Bekanntmachung des Hinweises auf die Patenterteilung: **21.11.84 Patentblatt 84/47**

(84) Benannte Vertragsstaaten: **BE CH DE FR GB IT NL**

(56) Entgegenhaltungen:
**CH - A - 602 005**
**DE - A - 2 730 515**
**DE - A - 2 738 150**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Fuchs, Rainer, Dr., Roeberstrasse 8, D-5600 Wuppertal 1 (DE)**
Erfinder: **Hoffmann, Helmut, Prof. Dr., Tersteegenweg 17, D-5600 Wuppertal 1 (DE)**
Erfinder: **Hammann, Ingeborg, Dr., Belfortstrasse 9, D-5000 Köln 80 (DE)**
Erfinder: **Homeyer, Bernhard, Dr., Obere Strasse 28, D-5090 Leverkusen 3 (DE)**
Erfinder: **Stendel, Wilhelm, Dr., In den Birken 55, D-5600 Wuppertal 1 (DE)**

EP 0 009 708 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue substituierte Bromostyrylcyclopropancarbonsäure-phenoxybenzylester, Verfahren und Zwischenprodukte zu ihrer Herstellung sowie ihre Verwendung als Insektizide und Akarizide.

Es ist bekannt, daß bestimmte Styryl-cyclopropancarbonsäure-phenoxybenzylester, wie z. B. 3-(2-Phenyl-vinyl)- und 3-(2-Chloro-2-phenyl-vinyl)-2,2-dimethyl-cyclopropancarbonsäure-(3-phenoxybenzyl)-ester, insektizid und akarizid wirksam sind (vergleiche DE-OS 2 738 150). Die Wirkung dieser Verbindungen ist jedoch, insbesondere bei niedrigen Wirkstoffkonzentrationen und Aufwandmengen, nicht immer zufriedenstellend.

Gegenstand der vorliegenden Erfindung sind

(1) neue substituierte Bromostyryl-cyclopropancarbonsäure-phenoxybenzylester der Formel I

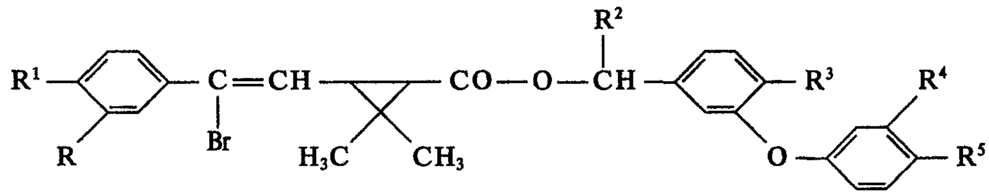

(I)

in welcher

R für Wasserstoff, Halogen, Alkyl oder Alkoxy steht,

$R^1$ für Wasserstoff, Halogen, Alkyl, Alkoxy, Alkylthio, oder zusammen mit R für Methylendioxy steht,

$R^2$ für Wasserstoff, Cyano oder Äthinyl steht,

$R^3$, $R^4$ und $R^5$ gleich oder verschieden sind und für Wasserstoff oder halogen stehen mit der Maßgabe, daß wenigstens einer der Reste $R^3$, $R^4$ oder $R^5$ für Halogen steht;

(2) ein Verfahren zur Herstellung von Bromostyrylcyclopropancarbonsäure-phenoxybenzylestern der Formel I, das dadurch gekennzeichnet ist, daß man Bromostyryl-cyclopropancarbonsäuren der Formel II

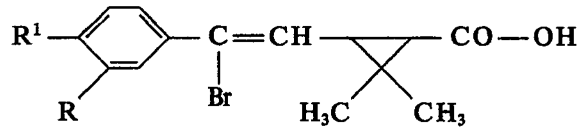

(II)

in welcher

R und $R^1$ die unter (1) angegebene Bedeutung haben, oder reaktionsfähige Derivate derselben mit substituierten Phenoxybenzylalkoholen der Formel III

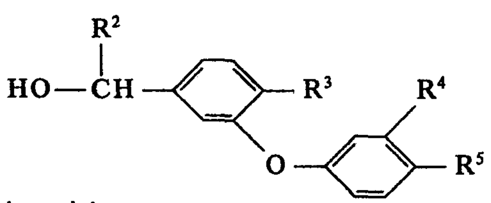

(III)

in welcher

$R^2$, $R^3$, $R^4$ und $R^5$ die oben angegebene Bedeutung haben, oder mit reaktionsfähigen Derivaten derselben gegebenenfalls in Gegenwart eines Säureakzeptors und gegebenenfalls unter Verwendung eines oder mehrerer Verdünnungsmittel umsetzt;

(3) neue Bromostyryl-cyclopropancarbonsäuren der Formel II

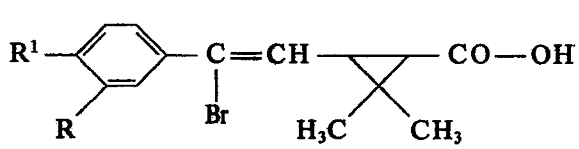

(II)

in welcher

R und $R^1$ die unter (1) angegebene Bedeutung haben;

(4) ein Verfahren zur Herstellung von Bromostyrylcyclopropancarbonsäuren der Formel II, dadurch gekennzeichnet, daß man α-Bromo-benzyl-phosphonsäureester der Formel IV

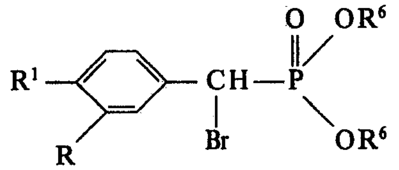

(IV)

in welcher
R und $R^1$ die unter (1) angegebene Bedeutung haben und
$R^6$ für Alkyl oder Phenyl steht oder die beiden Reste $R^6$ zusammen für Alkandiyl stehen,

mit Formyl-cyclopropancarbonsäureestern der Formel V

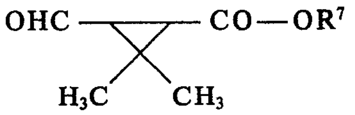

(V)

in welcher
$R^7$ für Alkyl steht,

in Gegenwart von Basen und gegebenenfalls unter Verwendung von Verdünnungsmitteln umsetzt und die dabei gebildeten Bromostyryl-cyclopropancarbonsäureester nach bekannten Methoden, beispielsweise durch Erhitzen mit Natriumhydroxid in wäßrigem Alkohol zu den entsprechenden Bromostyryl-cyclopropancarbonsäuren der Formel II verseift;

(5) neue α-Bromo-benzyl-phosphonsäureester der Formel IVa

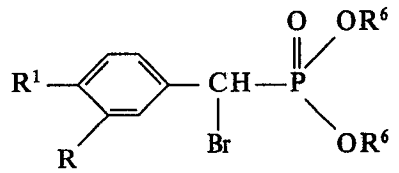

(IVa)

in welcher
R die unter (1) angegebene Bedeutung hat,
$R^1$ für Wasserstoff, Halogen, Alkoxy, Alkylthio oder zusammen mit R für Methylendioxy steht und
$R^6$ die unter (4) angegebene Bedeutung hat;

(6) ein Verfahren zur Herstellung von α-Bromo-benzylphosphonsäureestern der Formel IV, dadurch gekennzeichnet, daß man α-Hydroxy-benzyl-phosphonsäureester der Formel VI

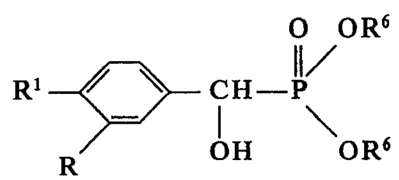

(VI)

in welcher
R, $R^1$ und $R^6$ die unter (1) bzw. (4) angegebene Bedeutung haben,
mit Dibrom-triphenylphosphoran, welches man gegebenenfalls in situ aus Triphenylphosphin und Brom erzeugt, gegebenenfalls in Gegenwart eines säureakzeptors und gegebenenfalls in Gegenwart eines Säureakzeptors und gegebenenfalls unter Verwendung eines Verdünnungsmittels umsetzt.

Die neuen Bromostyryl-cyclopropancarbonsäure-phenoxybenzylester der Formel (I) zeichnen sich durch hohe insektizide und akarizide Wirksamkeit aus.
Überraschenderweise zeigen die erfindungsgemäßen Bromostyryl-cyclopropancarbonsäure-phenoxybenzylester eine erheblich höhere insektizide und akarizide Wirkung als die aus dem Stand der Technik bekannten Verbindungen analoger Konstitution und gleicher Wirkungsrichtung.

Gegenstand der Erfindung sind vorzugsweise Bromostyrylcyclopropancarbonsäureester der Formel I, in welcher

R Für Wasserstoff, Fluor, Chlor, Brom, Methyl oder Methoxy steht,
$R^1$ für Wasserstoff, Fluor, Chlor, Brom, Methyl, Methoxy, Methylthio oder zusammen mit R für Methylendioxy steht,
$R^2$ für Wasserstoff, Cyano oder Äthinyl steht,
$R^3$, $R^4$ und $R^5$ gleich oder verschieden sind und für Wasserstoff oder Fluor stehen mit der Maßgabe, daß wenigstens einer der Reste $R^3$, $R^4$ oder $R^5$ für Fluor steht.

Die allgemeine Formel (I) schließt die verschiedenen möglichen Stereoisomeren und optisch aktiven Isomeren und deren Mischungen mit ein.

In einem bevorzugten Verfahren zur Herstellung der neuen Verbindungen der Formel (I) werden als reaktionsfähige Derivate der Bromostyryl-cyclopropancarbonsäuren der Formel (II) die entsprechenden Carbonsäurechloride der Formel VII

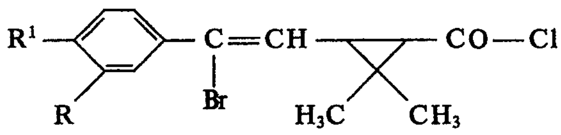
(VII)

in welcher
R und $R^1$ die oben angegebene Bedeutung haben,
mit substituierten Phenoxybenzylalkoholen der Formel III

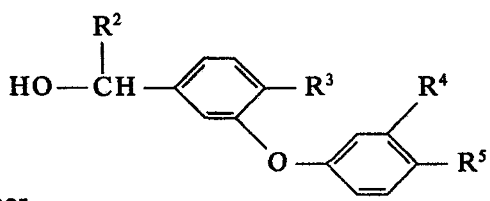
(III)

in welcher
$R^2$, $R^3$, $R^4$ und $R^5$ die oben angegebene Bedeutung haben,
gegebenenfalls in Gegenwart eines Säureakzeptors und gegebenenfalls unter Verwendung eines Verdünnungsmittels umgesetzt.

Ein besonders bevorzugtes Verfahren zur Herstellung von Verbindungen der Formel (I), worin $R^2$ für Cyano steht, ist dadurch gekennzeichnet, daß man Carbonsäurechloride der Formel VII (oben) mit substituierten Phenoxybenzaldehyden der Formel VIII

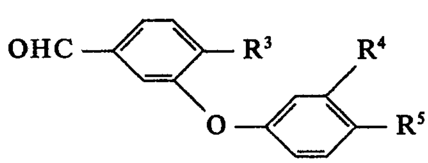
(VIII)

in welcher
$R^3$, $R^4$ und $R^5$ die oben angegebene Bedeutung haben, in Gegenwart von wenigstens der äquimolaren Menge Alkalicyanid (vorzugsweise Natrium- oder Kaliumcyanid), gegebenenfalls in Gegenwart eines Katalysators und gegebenenfalls unter Verwendung von Verdünnungsmitteln umsetzt.

Verwendet man beispielsweise bei der bevorzugten Verfahrensvariante (a) 3-(2-Bromo-2-(4-methoxy-phenyl)-vinyl)-2,2-dimethyl-cyclopropancarbonsäurechlorid und 3-(3-Fluoro-phenoxy)-benzylalkohol und bei Variante (b) 3-(2-Bromo-2-(3,4-dichloro-phenyl)-vinyl)-2,2-dimethyl-cyclopropancarbonsäurechlorid, Natriumcyanid und 3-(4-Fluoro-phenoxy)-4-fluoro-benzaldehyd als Ausgangsstoffe, so können die entsprechenden Reaktionen durch folgende Formelschemata skizziert werden:

(a)

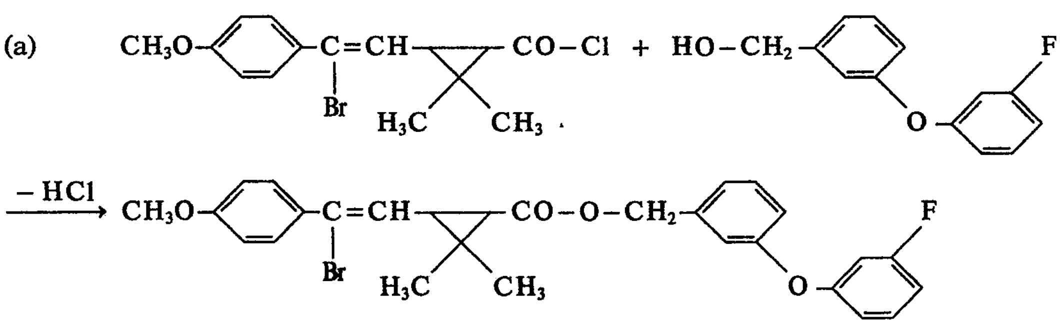

(b)

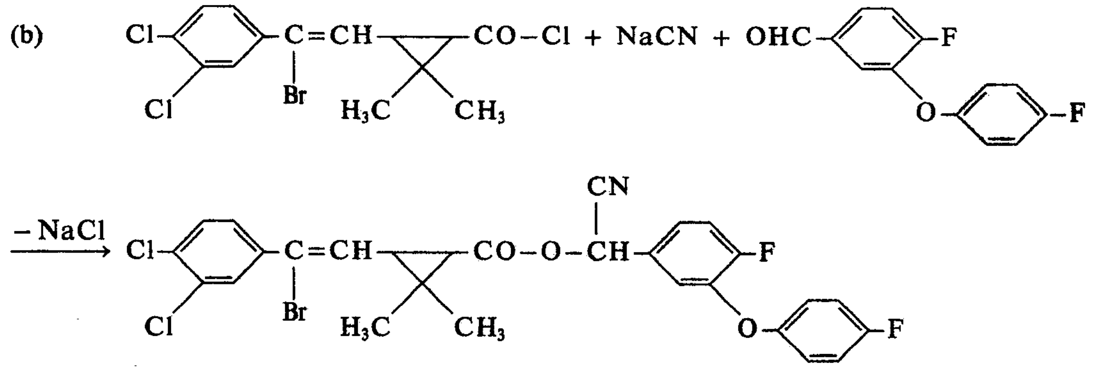


Die zu verwendenden Ausgangsstoffe sind durch die Formeln (II), (III), (VII) und (VIII) definiert. Vorzugsweise stehen darin die Reste R bis $R^5$ für diejenigen Reste, welche bei der Definition der Reste R bis $R^5$ in Formel (I) als bevorzugt genannt wurden.

Die als Ausgangsverbindungen zu verwendenden Bromostyryl-cyclopropancarbonsäuren (II) bzw. die entsprechenden Säurechloride (VII) als deren reaktionsfähige Derivate sind neu.

Man erhält die Carbonsäuren der Formel (II) aus den entsprechenden Alkylestern, vorzugsweise den Methyl- oder Äthylestern, durch Verseifung d. h. durch mehrstündiges Erhitzen auf Temperaturen zwischen 50 und 150°C, beispielsweise mit Natriumhydroxid in wäßrigem Alkohol. Die Aufarbeitung erfolgt auf übliche Weise, z. B. durch Abziehen des Alkohols im Vakuum, Verdünnen mit Wasser, Ansäuern der wäßrigen Phase, Extrahieren mit Methylenchlorid, Trocknen der organischen Phase und Abziehen des Lösungsmittels.

Die den Carbonsäuren der Formel (II) entsprechenden Ester erhält man, wie oben unter (4) erläutert, durch Umsetzung von $\alpha$-Bromo-benzyl-phosphonsäureestern der Formel IV (oben) mit Formyl-cyclopropancarbonsäureestern der Formel V (oben) in Gegenwart einer Base wie z. B. Natriummethylat und gegebenenfalls unter Verwendung von Verdünnungsmitteln wie z. B. Äthanole und Tetrahydrofuran, bei Temperaturen zwischen –10 und +50°C. Die Aufarbeitung kann auf übliche Weise durchgeführt werden, z. B. indem man das Reaktionsgemisch mit Wasser verdünnt, mit Methylenchlorid extrahiert, die organische Phase trocknet, das Lösungsmittel abzieht und das zurückbleibende Produkt im Vakuum destilliert.

Die den Carbonsäuren der Formel (II) entsprechenden Säurechloride der Formel (VII) können durch Umsetzung der Carbonsäuren (II) mit einem Halogenierungsmittel wie z. B. Thionylchlorid, gegebenenfalls unter Verwendung eines Verdünnungsmittels wie z. B. Tetrachlorkohlenstoff, bei Temperaturen zwischen 10 und 100°C hergestellt und durch Vakuumdestillation gereinigt werden.

Als Beispiele für die Carbonsäuren der Formel (II) sowie für die entsprechenden Säurechloride (VII) und Ester seien genannt:

3-(2-Bromo-2-phenyl-vinyl)-, 3-(2-Bromo-2-(4-fluorophenyl)-vinyl-, 3-(2-Bromo-2-(4-chloro-phenyl)-vinyl)-, 3-(2-Bromo-2-(3-bromo-phenyl)-vinyl-, 3-(2-Bromo-2-(4-bromo-phenyl)-vinyl)-, 3-(2-Bromo-2-(3-methyl-phenyl)-vinyl)-, 3-(2-Bromo-2-(4-methyl-phenyl)-vinyl-, 3-(2-Bromo-2-(3-methoxy-phenyl)-vinyl)-, 3-(2-Bromo-2-(4-methylthio-phenyl)-vinyl)-, 3-(2-Bromo-2-(3,4-dichlorophenyl)-vinyl)-, 3-(2-Bromo-2-(3,4-dimethyl-phenyl)-vinyl)-, 3-(2-Bromo-2-(3,4-dimethoxy-phenyl)-vinyl- und 3-(2-Bromo-2-(3,4-methylendioxy-phenyl)-vinyl-2,2-dimethyl-cyclopropancarbonsäure sowie die entsprechenden Säurechloride, die Methylester und die Äthylester.

Die als Zwischenprodukte zu verwendenden $\alpha$-Bromo-benzylphosphonsäureester sind durch Formel (IV) definiert. Vorzugsweise stehen darin

R und $R^1$ für diejenigen Reste, welche bei der Definition der Reste R und $R^1$ in Formel (I) als bevorzugt genannt sind und

$R^6$ steht vorzugsweise für Methyl, Äthyl, Phenyl oder beide Reste $R^6$ stehen zusammen für 2,2-Dimethyl-1,3-propandiyl.

Als Beispiele seien genannt:

$\alpha$-Bromo-benzyl-, $\alpha$-Bromo-4-fluoro-benzyl-, $\alpha$-Bromo-4-chloro-benzyl-, $\alpha$-Bromo-3-bromo-benzyl-, $\alpha$-Bromo-4-bromo-benzyl-, $\alpha$-Bromo-3-methoxy-benzyl-, $\alpha$-Bromo-4-methoxy-benzyl-, $\alpha$-Bromo-4-methylthio-benzyl-, $\alpha$-Bromo-3,4-dichloro-benzyl-, $\alpha$-Bromo-3,4-dimethoxy-benzyl- und $\alpha$-Bromo-3,4-methylendioxy-benzyl-phosphonsäure-dimethylester, -phosphonsäure-diäthylester und -phosphonsäurediphenylester.

Die Verbindungen der Formel (IV) sind teilweise bekannt ($R^1$ = Alkyl; vergleiche Zh: Obsc. Khim. 39 (1969), 1253—1256).

Man erhält die α-Bromo-benzyl-phosphonsäureester der Formel (IV), wie oben unter (6) erläutert, durch Umsetzung von α-Hydroxy-benzyl-phosphonsäureestern der Formel VI (oben) mit Dibrom-triphenylphosphoran, welches man gegebenenfalls in situ aus Triphenylphosphin und Brom erzeugt, gegebenenfalls in Gegenwart eines Säureakzeptors wie z. B. Pyridin, gegebenenfalls unter Verwendung eines Verdünnungsmittels wie z. B. Methylenchlorid bei Temperaturen zwischen —50 und +50°C (vergleiche Houben—Weyl, Methoden der organischen Chemie, 4. Auflage (1960, Band 5/4, S. 404—405, Thieme Verlag, Stuttgart).

Die Aufarbeitung kann nach üblichen Methoden durchgeführt werden, beispielsweise indem man das Verdünnungsmittel im Vakuum abdestilliert, den Rückstand mit Äther digeriert, vom ungelösten Triphenylphosphinoxid absaugt und den Äther im Vakuum abdestilliert. Das zurückbleibende Rohprodukt kann durch Vakuumdestillation gereinigt werden.

Die α-Hydroxy-benzyl-phosphonsäureester, welche als Zwischenprodukte verwendet werden, sind durch Formel VI (oben) definiert. Vorzugsweise stehen darin R, $R^1$ und $R^6$ für diejenigen Reste, welche bereits bei der Definition der Reste R, $R^1$ und $R^6$ in Formel (IV) als bevorzugt genannt wurden:

Als Beispiele seien genannt:

α-Hydroxy-benzyl-, α-Hydroxy-4-fluoro-benzyl-, α-Hydroxy-4-chloro-benzyl-, α-Hydroxy-4-bromo-benzyl-, α-Hydroxy-3-bromo-benzyl-, α-Hydroxy-3-methoxy-benzyl-, α-Hydroxy-4-methoxy-benzyl-, α-Hydroxy-4-methylthiobenzyl-, α-Hydroxy-3,4-dichloro-benzyl-, α-Hydroxy-3,4-dimethoxy-benzyl- und α-Hydroxy-3,4-methylendioxy-benzyl-phosphonsäuredimethylester, -phosphonsäurediäthylester und -phosphonsäurediphenylester.

Die α-Hydroxy-benzyl-phosphonsäureester der Formel VI sind teilweise bekannt. Man erhält sie im allgemeinen durch Umsetzung von Aldehyden der Formel IX

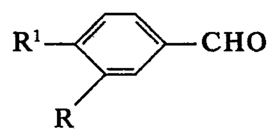

(IX)

in welcher
R und $R^1$ die oben angegebene Bedeutung haben,
mit Phosphorigsäureestern der Formel X

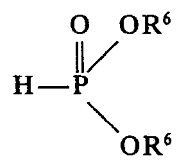

(X)

in welcher
$R^6$ die oben angegebene Bedeutung hat,

gegebenenfalls in Gegenwart eines Katalysators wie z. B. Triäthylamin, bei Temperaturen zwischen 10 und 100°C (vergleiche Houben—Weyl, Methoden der organischen Chemie, 4. Auflage (1963), Band 12/1, S. 475—483, Thieme Verlag, Stuttgart).

Als Beispiele für die Aldehyde der Formel (IX), welche bekannte Verbindungen sind, seien genannt:

Benzaldehyd, 4-Fluoro-benzaldehyd, 4-Chloro-benzaldehyd, 4-Bromo-benzaldehyd, 3-Bromo-benzaldehyd, 3-Methoxybenzaldehyd, 4-Methoxy-benzaldehyd, 3,4-Dichloro-benzaldehyd, 3,4-Dimethoxy-benzaldehyd und 3,4-Methylendioxy-benzaldehyd.

Als Beispiele für die Phosphorigsäureester der Formel (X), welche ebenfalls bekannte Verbindungen sind, seien genannt:

Phosphorigsäure-dimethylester (Dimethylphosphit),
Phosphorigsäure-diäthylester (Diäthylphosphit) und
Phosphorigsäure-diphenylester (Diphenylphosphit).

Die weiter als Zwischenprodukte zu verwendenden Formylcyclopropancarbonsäureester sind durch Formel (V) definiert. Vorzugsweise steht darin

$R^7$ für geradkettiges oder verzweigtes Alkyl mit 1 bis 4 Kohlenstoffatomen.

Als Beispiele seien genannt:

2,2-Dimethyl-3-formyl-cyclopropancarbonsäure-methylester, -äthylester, -n-propylester, -iso-propylester, -n-butylester, -iso-butylester, -sec.-butylester und -tert.-butylester.

Die Formyl-cyclopropancarbonsäureester der Formel (V) sind bekannt oder können nach bekannten Verfahren, im allgemeinen durch Umsetzung von bekannten Alken(I)ylcyclopropancarbonsäureestern mit Ozon hergestellt werden (vergleiche US-PS 3 679 667).
Die zur Herstellung der neuen Bromostyryl-cyclopropancarbonsäure-phenoxybenzylester (I) als Ausgangsverbindungen zu verwendenden Phenoxybenzylalkohole der Formel (III) und die entsprechenden Phenoxybenzaldehyde der Formel (VIII) sind bekannt oder können analog bekannten Verfahren hergestellt werden (vergleiche DE-OS 2 615 435 und 2 709 264).

Als Beispiele seien genannt:

3-(3-Fluoro-phenoxy)-, 3-(4-Fluoro-phenoxy)-, 3-Phenoxy-4-fluoro-,
3-(Fluoro-phenoxy)-4-fluoro- und 3-(4-Fluorophenoxy)-4-fluoro-benzylalkohol,
3-(3-Fluoro-phenoxy)-, 3-(4-Fluoro-phenoxy)-, 3-Phenoxy-4-fluoro-,
3-(3-Fluoro-phenoxy)-4-fluoro- und 3-(4-Fluorophenoxy)-4-fluoro-$\alpha$-cyano-benzylalkohol,
3-(3-Fluoro-phenoxy)-, 3-(4-Fluoro-phenoxy)-, 3-Phenoxy-4-fluoro-,
3-(3-Fluoro-phenoxy)-4-fluoro- und 3-(4-Fluorophenoxy)-4-fluoro-$\alpha$-äthinyl-benzalkohol sowie
3-(3-Fluoro-phenoxy)-, 3-(4-Fluoro-phenoxy)-, 3-Phenoxy-4-fluoro-,
3-(3-Fluoro-phenoxy)-4-fluoro- und 3-(4-Fluorophenoxy)-4-fluoro-benzaldehyd.

Das Verfahren zur Herstellung der erfindungsgemäßen Bromostyryl-cyclopropancarbonsäure-phenoxybenzylester wird in allen Varianten bevorzugt unter Verwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.
Beim Arbeiten im Zweiphasenmedium wird Wasser als zweite Lösungsmittelkomponente verwendet. Bei dem als bevorzugt beschriebenen Verfahren, ausgehend von Säurechloriden der Formel (VII) und Phenoxybenzylalkoholen der Formel (III), können als Säureakzeptoren alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.
Bei dem als besonders bevorzugt beschriebenen Verfahren, ausgehend von Säurechloriden der Formel (VII) und Phenoxybenzaldehyden der Formel (VIII), werden als Katalysatoren im allgemeinen Verbindungen verwendet, welche gewöhnlich bei Reaktionen in mehrphasigen Medien als Hilfsmittel zum Phasentransfer von Reaktanden dienen. Insbesondere seien Tetraalkyl- und Trialkyl-aralkylammoniumsalze wie z. B. Tetrabutylammoniumbromid und Trimethyl-benzyl-ammoniumchlorid genannt.
Die Reaktionstemperatur kann innerhalb eines größeren Bereichs variiert werden. Im allgemeinen arbeitet man zwischen 0 und 100°C, vorzugsweise bei 10 bis 50°C.
Das erfindungsgemäße Verfahren wird im allgemeinen bei Normaldruck durchgeführt.
Zur Durchführung des erfindungsgemäßen Verfahrens werden die Ausgangsstoffe gewöhnlich in äquimolaren Mengen eingesetzt. Ein Überschuß der einen oder anderen Reaktionskomponente bringt keine wesentlichen Vorteile. Die Umsetzung wird im allgemeinen in einem oder mehreren Verdünnungsmitteln in Gegenwart eines Säureakzeptors oder eines Katalysators durchgeführt und das Reaktionsgemisch wird mehrere Stunden bei der erforderlichen Temperatur gerührt. Anschließend wird das Reaktionsgemisch mit Toluol/Wasser geschüttelt, die organische Phase abgetrennt, mit Wasser gewaschen und getrocknet.
Nach Abdestillieren des Lösungsmittels im Vakuum fallen im allgemeinen die neuen Verbindungen in Form von Ölen an, die sich zum Teil nicht unzersetzt destillieren lassen, jedoch durch sogenanntes ›Andestillieren‹, d. h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.
Die neuen Bromostyryl-cyclopropancarbonsäure-phenoxybenzylester zeichnen sich, wie bereits erwähnt, durch eine hohe insektizide und akarizide Wirksamkeit aus. Sie können gegen pflanzenschädigende Insekten und Milben in Land- und Forstwirtschaft, im Vorratsschutz und Hygienebereich sowie gegen Ektoparasiten im veterinärmedizinischen Bereich eingesetzt werden.

## Beispiel A

### Phaedon-Larven-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon chochleariae) besetzt, solange die Blätter noch feucht sind.

Nach der gewünschten Zeit wird die Abtötung in %bestimmt. Dabei bedeutet 100%, daß alle Käfer-Larven abgetötet wurden; 0% bedeutet, daß keine Käfer-Larven abgetötet wurden.

Bei diesem Test zeigen z. B. die folgenden Verbindungen der Herstellungsbeispiele überlegene Wirksamkeit gegenüber dem Stand der Technik: 4, 5, 2, 1.

## Beispiel B

### Tetranychus-Test (esistent)

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in %bestimmt. Dabei bedeutet 100%, daß alle Spinnmilben abgetötet wurden; 0% bedeutet, daß keine Spinnmilben abgetötet wurden.

Bei diesem Test zeigen z. B. die folgenden Verbindungen der Herstellungsbeispiele überlegene Wirksamkeit gegenüber dem Stand der Technik: 4, 5, 2, 1.

## Beispiel C

### Grenzkonzentrations-Test/Bodeninsekten

Testinsekt: Phorbia antiqua (im Boden)
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in %bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 4, 5, 3, 2, 1.

## Beispiel D

### Test mit parasitierenden Fliegenlarven (Lucilia cuprina res.)

Emulgator: 80 Gewichtsteile Alkylarylpolyglykoläther

Zur Herstellung einer zweckmäßigen Wirkstoffzubereitung vermischt man 20 Gew.-Teile der betreffenden aktiven Substanz mit der angegebenen Menge des Emulgators und verdünnt das so erhaltene Gemisch mit Wasser auf die gewünschte Konzentration. Etwa 20 Fliegenlarven (Lucilia cuprina) werden in ein mit Wattestopfen entsprechender Größe beschicktes Teströhrchen gebracht, welches ca. 3 ml einer 20%igen Eigelbpulver-Suspension in Wasser enthält. Auf diese Eigelbpulver-Suspension werden 0,5 ml der Wirkstoffzubereitung gebracht. Nach 24 Stunden wird der Abtötungsgrad bestimmt.

Bei diesem Test zeigen z. B. die folgenden Verbindungen der Herstellungsbeispiele überlegene Wirkung gegenüber dem Stand der Technik: 4, 5, 3, 1.

## Beispiel E

Test mit parasitierenden adulten Rinderzecken (Boophilus microplus res.)

Lösungsmittel: Alkylarylpolyglykoläther

Zur Herstellung einer zweckmäßigen Wirkstoffzubereitung vermischt man die betreffende aktive Substanz mit dem angegebenen Lösungsmittel im Verhältnis 1 : 2 und verdünnt das so erhaltene Konzentrat mit Wasser auf die gewünschte Konzentration.

10 adulte Rinderzecken (b. microplus res.) werden 1 Minute in die zu testende Wirkstoffzubereitung getaucht. Nach Überführung in Plastikbecher und Aufbewahrung in einem klimatisierten Raum wird der Abtötungsgrad bestimmt.

Bei diesem Test zeigen z. B. die folgenden Verbindungen der Herstellungsbeispiele überlegene Wirkung gegenüber dem Stand der Technik: 4, 5, 3, 1.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.
Aus der Ordnung der Diplopoda z. B. Blaniulus guttulatus.
Aus der Ordnung der Chilopoda z. B. Geophilus carpophagus, Scutigera spec.
Aus der Ordnung der Symphyla z. B. Scutigerella immaculata.
Aus der Ordnung der Thysanura z. B. Lepisma saccharina.
Aus der Ordnung der Collembola z. B. Onychiurus armatus.
Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z. B. Forficula auricularia.
Aus der Ordnung der Isoptera z. B. Reticulitermes spp.
Aus der Ordnung der Anoplura z. B. Phylloxera vastatrix, Pemphigus spp., Pediculus humanus corporis, Haematopinus spp., Linognathus spp.
Aus der Ordnung der Mallophaga z. B. Trichodectes spp., Damalinea ssp.
Aus der Ordnung der Thysanoptera z. B. Hercinothrips femoralis, Thrips tabaci.
Aus der Ordnung der Heteroptera z. B. Eurygaster spp., Dysdercus intermedius, Piesma quadrata, Cimex lectularius, Rhodnius prolixus, Triatoma spp.
Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus sp., Phorodon humuli, Rhopolosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Ato-

maria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma ssp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z. B. Diprion spp., Hoplocampa spp., Lasius spp., Monomorium pharaonis, Vespa spp.
Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratits capitata, Dacus oleae, Tipula paludosa.
Aus der Ordnung der Siphonaptera z. B. Xenopsylla cheopis, Ceratophyllus spp.,
Aus der Ordnung der Arachnida z. B. Scorpio maurus, Latrodectus mactans.
Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

## Herstellungsbeispiele

### Beispiel 1

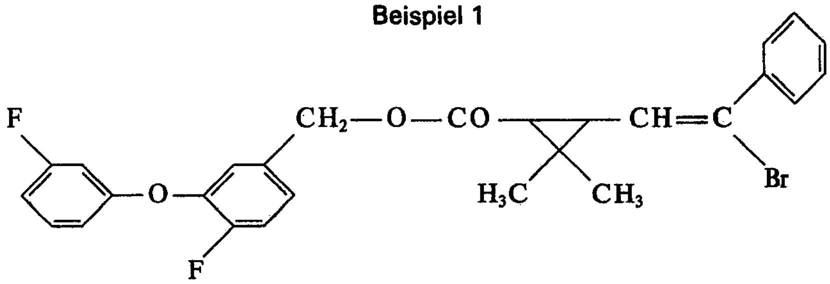

4,7 g (0,02 Mol) 3-(3-Fluoro-phenoxy)-4-fluoro-benzylalkohol und 6,3 g (0,02 Mol) 2,2-Dimethyl-3-(2-bromo-2-phenyl)-vinyl)-cyclopropancarbonsäurechlorid werden in 100 ml wasserfreiem Toluol gelöst und bei 20–25°C 2,5 g Pyridin, gelöst in 50 ml wasserfreiem Toluol, unter Rühren zugetropft. Anschließend wird weitere 3 Stunden bei 25–35°C gerührt. Das Reaktionsgemisch wird in 150 ml Wasser, dem 10 ml konzentrierte Salzsäure zugesetzt werden, gegossen, die organische Phase abgetrennt und nochmals mit 100 ml Wasser gewaschen. Anschließend wird die Toluolphase über Natriumsulfat getrocknet und das Lösungsmittel im Wasserstrahlvakuum abdestilliert. Letzte Lösungsmittelreste werden durch kurzes Andestillieren bei 60°C/l Torr Badtemperatur entfernt. Man erhält 7,5 g (75,5% der Theorie) 2,2-Dimethyl-3-(2-bromo-2-phenyl-vinyl)-cyclopropancarbonsäure-(4-fluoro-3-(3-fluorophenoxy)-benzyl)-ester als gelbes Öl mit dem Brechungsindex $n_D^{23}$: 1,5758.

### Beispiel 2

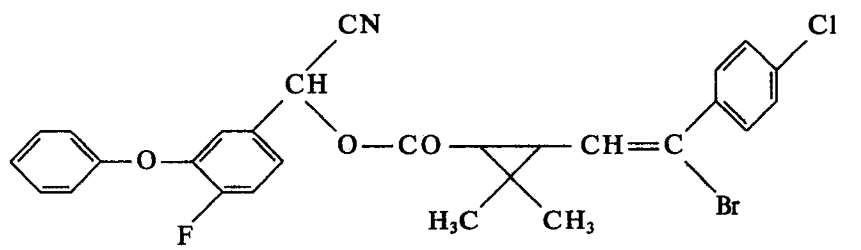

4,32 g (0,02 Mol) 3-Phenoxy-4-fluoro-benzaldehyd und 6,96 g (0,02 Mol) 2,2-Dimethyl-3-(2-bromo-2-(4-chlorophenyl)-vinyl)-cyclopropancarbonsäurechlorid werden zusammen unter Rühren bei 20–25°C zu einer Mischung von 1,6 g Natriumcyanid, 2,5 ml Wasser, 100 ml n-Hexan und 0,5 g Tetrabutylammoniumbromid getropft und dann 4 Stunden bei 20–25°C gerührt. Anschließend wird die Reaktionsmischung mit 300 ml Toluol versetzt und 2mal mit je 300 ml Wasser ausgeschüttelt. Die organische Phase wird abgetrennt, über Magnesiumsulfat getrocknet und das Lösungsmittel im Wasserstrahlvakuum abdestilliert. Letzte Lösungsmittelreste werden durch kurzes Andestillieren bei 60° C/l Torr Badtemperatur entfernt. Man erhält 7,8 g (70,3% der Theorie) 2,2-Dimethyl-3-(2-bromo-2-(4-chloro-phenyl)-vinyl)-cyclopropancarbonsäure-3-phenoxy-4-fluoro-$\alpha$-cyano-benzylester als zähes Öl. Die Struktur wird durch das $^1$H-NMR-Spektrum bestätigt. $^1$H-NMR-Spektrum ($CDCl_3$/TMS):

aromatische-H: 7,65–6,8 $\delta$ (m/12 H), benzyl-H: 6,5–6,3 $\delta$ (m/l H), vinyl-H: 6,6–6,4 $\delta$ und 6,1–5,9 $\delta$ (m/l H) cyclopropan-H: 2,7–1,5 $\delta$ (m/2 H), dimethyl-H: 1,5–1,1 $\delta$ (m/6 H).

Analog einem der Beispiele 1 und/oder 2 können die folgenden Verbindungen hergestellt werden:

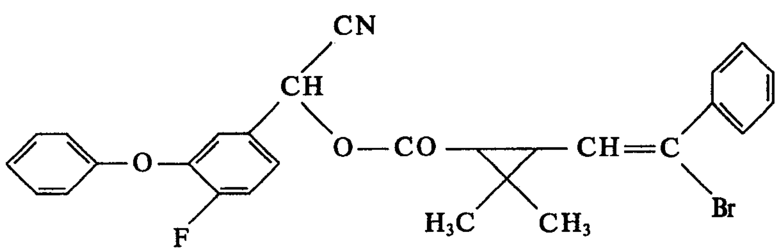

Ausbeute: 73,1 % der Theorie; Brechungsindex $n_D^{23}$: 1,5799

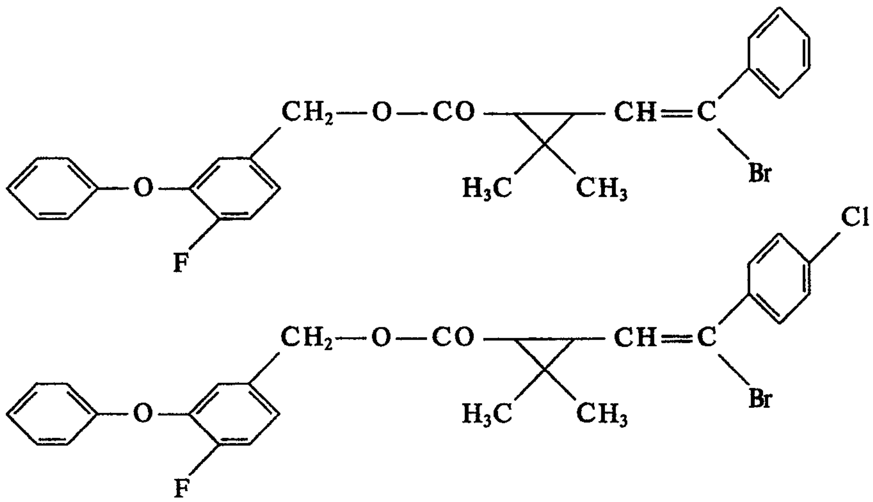

Ausbeute: 85,8 % der Theorie; Brechungsindex $n_D^{23}$: 1,5882

Ausbeute: 74,6 % der Theorie; Brechungsindex $n_D^{23}$: 1,5943

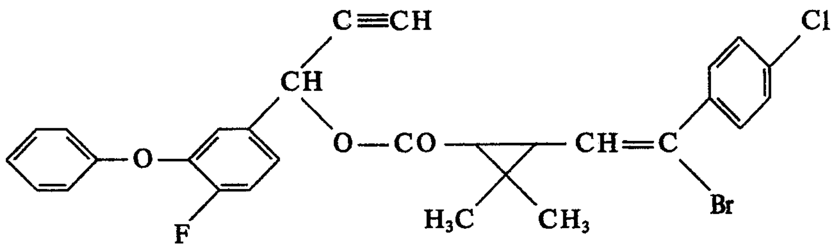

Die Ausgangsverbindungen können wie folgt hergestellt werden:

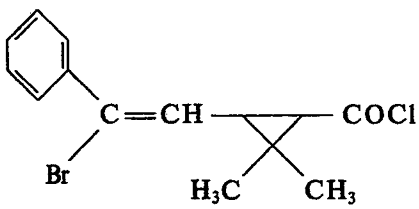

59 g (0,2 Mol) 2,2-Dimethyl-3-(2-bromo-2-(4-chloro-phenyl)-vinyl)-cyclopropancarbonsäure werden in 500 ml Tetrachlorkohlenstoff gelöst und bei 25°C langsam unter Rühren 119 g Thionylchlorid zugetropft. Anschließend wird 4 Stunden zum Rückfluß erhitzt. Nach beendeter Reaktionszeit werden überschüssiges Thionylchlorid sowie Tetrachlorkohlenstoff im Wasserstrahlvakuum abdestilliert. Das verbleibende Öl wird destilliert. Man erhält 33,7 g (53,8 % der Theorie) 2,2-Dimethyl-3-(2-bromo-2-(4-chloro-phenyl)-vinyl)-cyclopropancarbonsäurechlorid als farblose Flüssigkeit mit dem Siedepunkt 165–166°C bei 8 Torr.

Analog erhält man

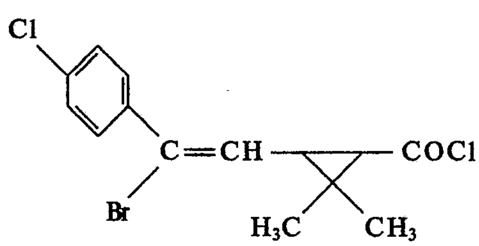

in einer Ausbeute von 92,5 % der Theorie.

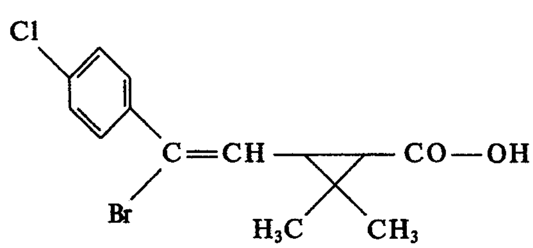

50 g (0,14 Mol) 2,2-Dimethyl-3-(2-bromo-2-(4-chloro-phenyl)-vinyl)-cyclopropancarbonsäure-äthylester werden in 100 ml Äthanol gelöst, dann mit einer Lösung von 6 g (0,15 Mol) Natriumhydroxid in 100 ml Wasser versetzt und 4 Stunden unter Rühren zum Rückfluß erhitzt. Anschließend wird das Äthanol im Wasserstrahlvakuum abdestilliert, der Rückstand in 300 ml warmem Wasser aufgenommen und 1 mal mit 300 ml Methylenchlorid extrahiert. Die wäßrige Phase wird abgetrennt, mit konzentrierter Salzsäure angesäuert und anschließend mit 2mal 300 ml Methylenchlorid extrahiert. Die organische Phase wird dann abgetrennt über Magnesiumsulfat, getrocknet und das Lösungsmittel im Wasserstrahlvakuum abdestilliert. Letzte Lösungsmittelreste werden durch kurzes Andestillieren bei 2 Torr/60° C Badtemperatur entfernt. Man erhält dann 37,8 g (81,9 % der Theorie) 2,2-Dimethyl-3-(2-bromo-2-(4-chloro-phenyl)-vinyl)-cyclopropancarbonsäure (cis und trans, E und Z-Isomerengemisch) als sehr zähes gelbes Öl. Die Struktur wird durch das $^1$H-NMR-Spektrum bestätigt.

$^1$H-NMR (in $CDCl_3$/TMS):

aromatische-H: 2,43–2,86 $\tau$ (m, 4 H), vinyl-H: 3,33–3,57 $\tau$ und 3,9–4,16 $\tau$ (2 m, 1 H), cyclopropan-H: 7,33–8,45 $\tau$ (m/2 H) und dimethyl-H: 8,45–8,88 (m/6 H).

Analog erhält man

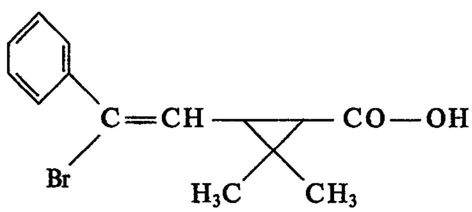

in einer Ausbeute von 78,6 % der Theorie.

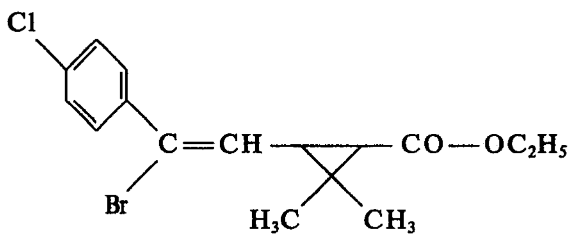

4,6 g (0,2 Mol) Natrium werden portionsweise in 100 ml Äthanol gelöst. Wenn sich alles Natrium aufgelöst hat, werden 100 ml Tetrahydrofuran (wasserfrei) zugesetzt und bei 0 °C unter Rühren 68,3 g (0,2 Mol) 4-Chloro-$\alpha$-bromo-benzyl-phosphonsäurediäthylester, gelöst in 50 ml wasserfreiem Tetrahydrofuran, zugetropft. Nachdem noch 2 Stunden bei 0–5 °C nachgerührt wurde, werden 34 g (0,2 Mol) cis/trans-2,2-Dimethyl-3-formyl-cyclopropancarbonsäureäthylester, gelöst in 50 ml wasserfreiem Tetrahydrofuran, unter Rühren bei 0 °C zugetropft. Anschließend wird noch 12 Stunden bei 20–25 °C nachgerührt. Die Reaktionsmischung wird dann mit 600 ml Wasser versetzt und 2mal mit je 300 ml Methylenchlorid extrahiert. Die organische Phase wird abgetrennt, über Magnesiumsulfat getrocknet, das Lösungsmittel im Wasserstrahlvakuum abdestilliert und der Rückstand im Vakuum destilliert. Man erhält 34,5 g (48,3 % der Theorie) 2,2-Dimethyl-3-(2-bromo-2-(4-chloro-phenyl)-vinyl)-cyclopropancarbonsäureäthylester (cis, trans und E, Z-Isomerengemisch) als gelbes Öl mit dem Siedepunkt 160–165 °C/l Torr.

Analog erhält man

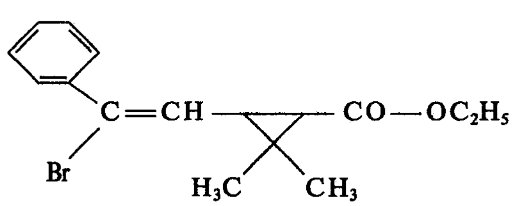

in einer Ausbeute von 50,2 % der Theorie mit dem Siedepunkt 140–150 °C/Torr.

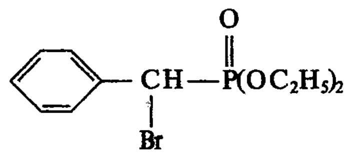

In eine Lösung von 131 g (0,5 Mol) Triphenylphosphin in 500 ml Methylenchlorid läßt man unter Feuchtigkeitsausschluß bei 30–35°C 90 g Brom gelöst in 250 ml Methylenchlorid zutropfen. Man rührt 1 Stunde bei Raumtemperatur nach, dann läßt man bei –20°C innerhalb 1 Stunde eine Lösung von 122 g (0,5 Mol) $\alpha$-Hydroxybenzyl-phosphonsäurediäthylester in 250 ml Methylenchlorid zutropfen, rührt 1 Stunde bei –20°C nach und tropft dann innerhalb von einer Stunde bei –20°C 40 g Pyridin, gelöst in 250 ml Methylenchlorid zu. Man läßt 20 Stunden nachrühren, wobei die Temperatur langsam bis +20°C ansteigt.

Das Reaktionsgemisch wird in einen Birnenkolben überführt und das Lösungsmittel im Wasserstrahlvakuum abdestilliert. Der Rückstand wird mit einem Liter Äther ausgerührt und von dem nicht gelösten Triphenylphosphinoxyd abgesaugt. (Triphenylphosphinoxyd 120 g = 86 % der Theorie). Die Mutterlauge wird im Vakuum eingeengt und der Rückstand im Hoxhvakuum destilliert. Man erhält 112 g (72 % der Theorie) $\alpha$-Brombenzyl-phosphonsäurediäthylester als hellgelbes Öl mit einem Siedepunkt von 110°C/0,01 Torr und einer Reinheit von 95,9 % (GC).

Analog erhält man:

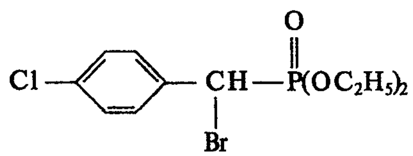

Ausbeute: 89,5 % der Theorie

Siedepunkt: 126° C/0,01 Torr

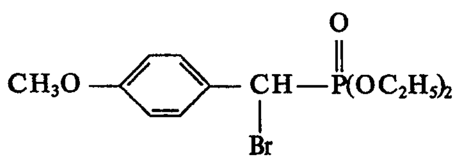

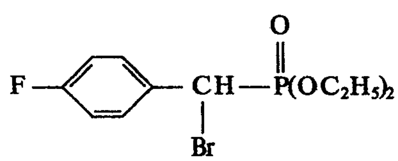

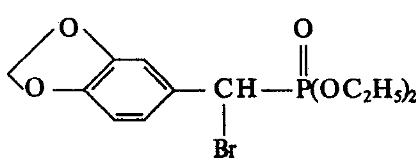

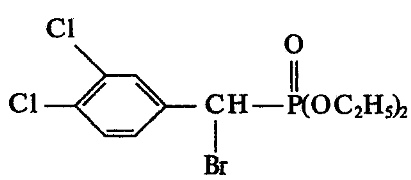

**Patentansprüche für die Vetragsstaaten: BE, CH, DE, FR, GB, NL.**

1. Substituierte Bromostyryl-cyclopropancarbonsäureester der Formel (I)

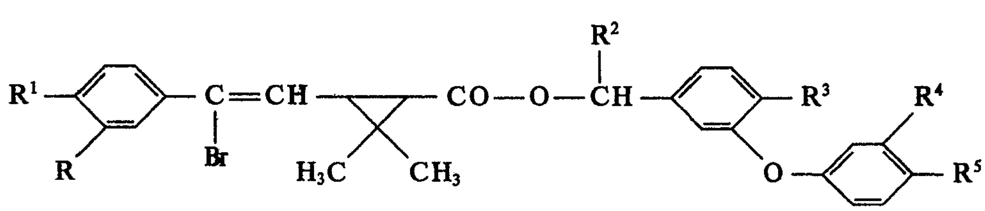

(I)

in welcher

R für Wasserstoff, Halogen, Alkyl oder Alkoxy steht,
$R^1$ für Wasserstoff, Halogen, Alkyl, Alkoxy, Alkylthio, oder zusammen mit R für Methylendioxy steht,
$R^2$ für Wasserstoff, Cyano oder Ethinyl steht,
$R^3$, $R^4$ und $R^5$ gleich oder verschieden sind und für Wasserstoff oder Halogen stehen mit der Maßgabe, daß wenigstens einer der Reste $R^3$, $R^4$ oder $R^5$ für Halogen steht.

2. Verfahren zur Herstellung von Bromostyryl-cyclopropancarbonsäure-phenoxybenzylestern der Formel I, gemäß Anspruch 1

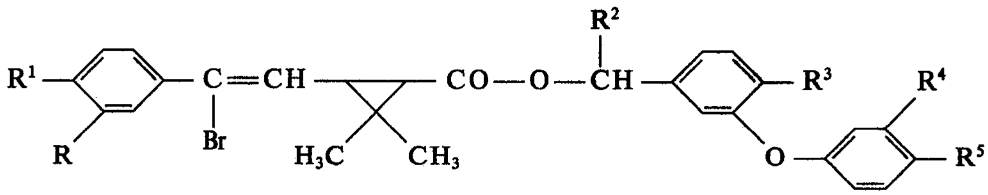

(I)

in welcher

R für Wasserstoff, Halogen, Alkyl oder Alkoxy steht,
$R^1$ für Wasserstoff, Halogen, Alkyl, Alkoxy, Alkylthio, oder zusammen mit R für Methylendioxy steht,
$R^2$ für Wasserstoff, Cyano oder Ethinyl steht,
$R^3$, $R^4$ und $R^5$ gleich oder verschieden sind und für Wasserstoff oder Halogen stehen mit der Maßgabe, daß wenigstens einer der Reste $R^3$, $R^4$ oder $R^5$ für Halogen steht,

dadurch gekennzeichnet, daß man Bromstyryl-cyclopropancarbonsäuren der Formel II

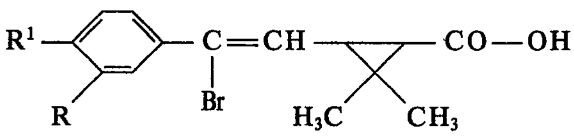

(II)

in welcher

R und $R^1$ die oben angegebenen Bedeutung haben, oder reaktionsfähige Derivate derselben mit substituierten Phenoxybenzylalkoholen der Formel III

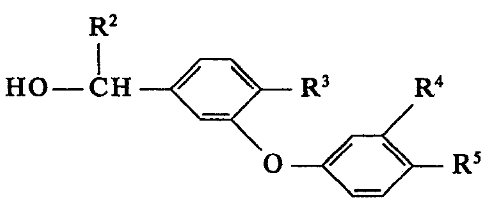

(III)

in welcher

$R^2$, $R^3$, $R^4$ und $R^5$ die oben angegebene Bedeutung haben,
oder mit reaktionsfähigen Derivaten derselben gegebenenfalls in Gegenwart eines Säureakzeptors und gegebenenfalls unter Verwendung eines oder mehrerer Verdünnungsmittel umsetzt.

3. Bromstyryl-cyclopropancarbonsäuren der Formel II

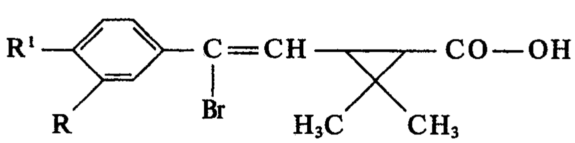

(II)

in welcher

R und $R^1$ die in Anspruch 1 angegebene Bedeutung haben mit Ausnahme der Verbindungen

3-[2-Phenyl-2-brom-vinyl]-, 3-[2-(4-Chlorphenyl)-2-brom-vinyl]-, 3-[2-(3,4-Dichlorphenyl)-2-brom-vinyl]-, 3-[2-(4-Fluorphenyl)-2-brom-vinyl]-, 3-[2-(4-tert.-Butylphenyl)-2-brom-vinyl]-2,2-dimethylcyclopropancarbonsäure bzw. -2,2-dimethylcyclopropancarbonsäurechlorid.

4. Insektizide und/oder akarizide Mittel, gekennzeichnet, durch einen Gehalt an mindestens einem substituierten Bromostyryl-cyclopropancarbonsäureester der Formel (I).

5. Verwendung von substituierten Bromostyryl-cyclopropancarbonsäureestern der Formel (I) zur Bekämpfung von Insekten und/oder Spinnentieren.

6. Verfahren zur Bekämpfung von Insekten und/oder Spinnentieren, dadurch gekennzeichnet, daß man substituierte Bromostyryl-cyclopropancarbonsäureester der Formel (I) auf Insekten und/oder Spinnentiere und/oder ihren Lebensraum einwirken läßt.

7. Verfahren zur Herstellung insektizider und/oder akarizider Mittel, dadurch gekennzeichnet, daß man substituierte Bromostyryl-cyclopropancarbonsäureester der Formel (I) mit Streckmitteln und/oder oberflächenaktiven Mitteln vermischt.

**Patentansprüche für den Vertragsstaat: It.**

1. Substituierte Bromostyryl-cyclopropancarbonsäureester der Formel (I)

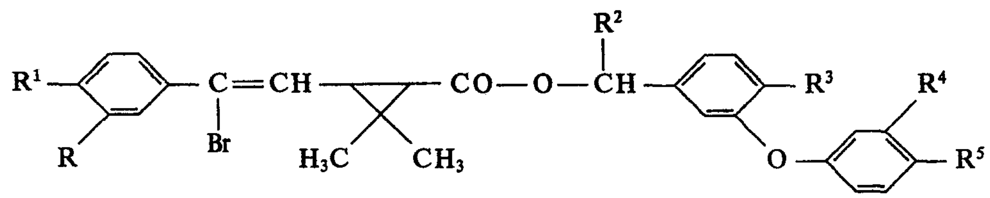

(I)

in welcher

R für Wasserstoff, Halogen, Alkyl oder Alkoxy steht,
$R^1$ für Wasserstoff, Halogen, Alkyl, Alkoxy, Alkylthio, oder zusammen mit R für Methylendioxy steht,
$R^2$ für Wasserstoff, Cyano oder Ethinyl steht,
$R^3$, $R^4$ und $R^5$ gleich oder verschieden sind und für Wasserstoff oder Halogen stehen mit der Maßgabe, daß wenigstens einer der Reste $R^3$, $R^4$ oder $R^5$ für Halogen steht.

2. Verfahren zur Herstellung von Bromostyryl-cyclopropancarbonsäure-phenoxybenzylestern der Formel I, gemäß Anspruch 1

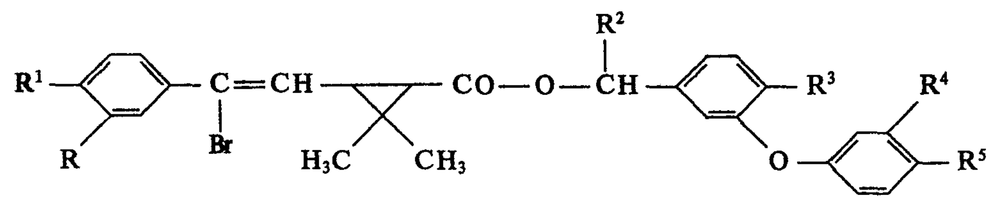

(I)

in welcher

R für Wasserstoff, Halogen, Alkyl oder Alkoxy steht,
$R^1$ für Wasserstoff, Halogen, Alkyl, Alkoxy, Alkylthio, oder zusammen mit R für Methylendioxy steht,
$R^2$ für Wasserstoff, Cyano oder Ethinyl steht,
$R^3$, $R^4$ und $R^5$ gleich oder verschieden sind und für Wasserstoff oder Halogen stehen mit der Maßgabe, daß wenigstens einer der Reste $R^3$, $R^4$ oder $R^5$ für Halogen steht,

dadurch gekennzeichnet, daß man Bromstyryl-cyclopropancarbonsäuren der Formel II

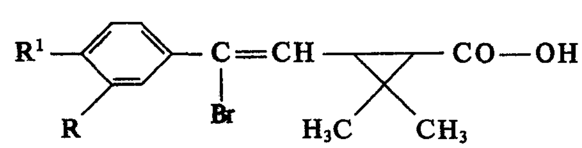

(II)

in welcher

R und $R^1$ die oben angegebenen Bedeutung haben, oder reaktionsfähige Derivate derselben mit substituierten Phenoxybenzylalkoholen der Formel III

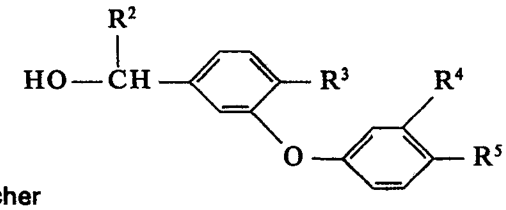

(III)

in welcher

$R^2$, $R^3$, $R^4$ und $R^5$ die oben angegebene Bedeutung haben,
oder mit reaktionsfähigen Derivaten derselben gegebenenfalls in Gegenwart eines Säureakzeptors und gegebenenfalls unter Verwendung eines oder mehrerer Verdünnungsmittel umsetzt.

3. Bromstyryl-cyclopropancarbonsäuren der Formel II

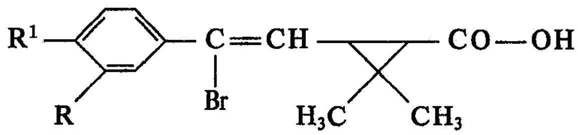

(II)

in welcher

R und $R^1$ die in Anspruch 1 angegebene Bedeutung haben.

4. Verfahren zur Herstellung von Bromostyryl-cyclopropancarbonsäuren der Formel II, dadurch gekennzeichnet, daß man $\alpha$-Bromo-benzyl-phosphonsäureester der Formel IV

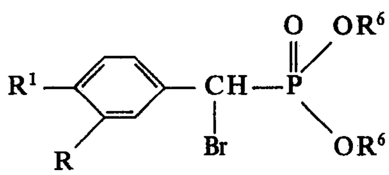

(IV)

in welcher

R und $R^1$ die in Anspruch 1 angegebene Bedeutung haben und
$R^6$ für Alkyl oder Phenyl steht oder die beiden Reste $R^6$ zusammen für Alkandiyl stehen,

mit Formyl-cyclopropancarbonsäureestern der Formel V

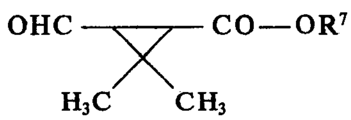

(V)

in welcher

$R^7$ für Alkyl steht,

in Gegenwart von Basen und gegebenenfalls unter Verwendung von Verdünnungsmitteln umsetzt und die dabei gebildeten Bromostyryl-cyclopropancarbonsäureester nach bekannten Methoden, beispielsweise durch Erhitzen mit Natriumhydroxid in wäßrigem Alkohol zu den entsprechenden Bromostyryl-cyclopropancarbonsäuren der Formel II verseift.

5. Insektizide und/oder akarizide Mittel, gekennzeichnet, durch einen Gehalt an mindestens einem substituierten Bromostyryl-cyclopropancarbonsäureester der Formel (I).

6. Verwendung von substituierten Bromostyryl-cyclopropancarbonsäureestern der Formel (I) zur Bekämpfung von Insekten und/oder Spinnentieren.

7. Verfahren zur Bekämpfung von Insekten und/oder Spinnentieren, dadurch gekennzeichnet, daß man substituierte Bromostyryl-cyclopropancarbonsäureester der Formel (I) auf Insekten und/oder Spinnentiere und/oder ihren Lebensraum einwirken läßt.

8. Verfahren zur Herstellung insektizider und/oder akarizider Mittel, dadurch gekennzeichnet, daß man substituierte Bromostyryl-cyclopropancarbonsäureester der Formel (I) mit Streckmitteln und/oder oberflächenaktiven Mitteln vermischt.

**Claims for the contracting states: BE, CH, DE, FR, GB, NL.**

1. Substituted bromostyryl-cyclopropanecarboxylic acid esters of the formula (I)

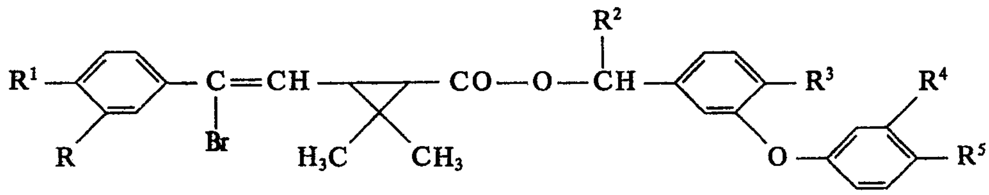

(I)

in which

R represents hydrogen, halogen, alkyl or alkoxy,
$R^1$ represents hydrogen, halogen, alkyl, alkoxy or alkylthio or, together with R, represents methylene-dioxy,
$R^2$ represents hydrogen, cyano or ethinyl and
$R^3$, $R^4$ und $R^5$ are identical or different and represent hydrogen or halogen, with the proviso that at least one of the radicals $R^3$, $R^4$ or $R^5$ represents halogen.

2. Process for the preparation of bromostyryl-cyclopropanecarboxylic acid phenoxybenzyl esters of the formula I to Claim 1,

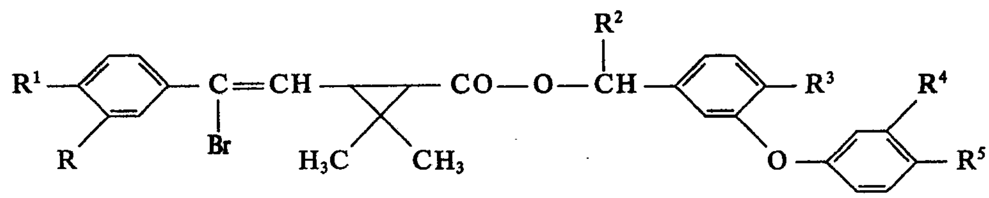

(I)

in which

R represents hydrogen, halogen, alkyl or alkoxy,
$R^1$ represents hydrogen, halogen, alkyl, alkoxy or alkylthio or, together with R, represents methylene-dioxy,
$R^2$ represents hydrogen, cyano or ethinyl and
$R^3$, $R^4$ and $R^5$ are identical or different and represent hydrogen or halogen, with the proviso that at least one of the radicals $R^3$, $R^4$ or $R^5$ represents halogen,

characterised in that bromostyryl-cyclopropanecarboxylic acids of the formula II

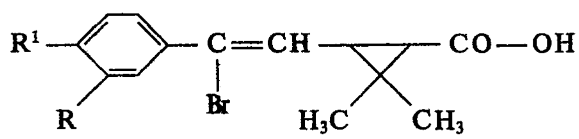

(II)

in which

R und $R^1$ have the meaning indicated above, or reactive derivatives thereof are reacted with substituted phenoxybenzyl alcohols of the formula III

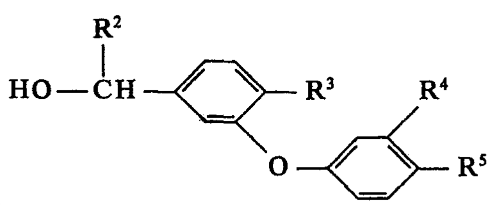

(III)

in which

$R^2$, $R^3$, $R^4$ and $R^5$ have the meaning indicated above, or with reactive derivatives thereof, if appropriate in the presence of an acid acceptor and if appropriate using one or more diluents.

3. Bromostyryl-cyclopropanecarboxylic acids of the formula II

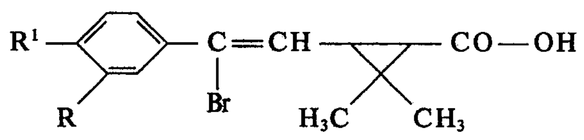

(II)

in which

R and $R^1$ have the meaning indicated in Claim 1, with the exeption o the compounds

3-[2-phenyl-2-bromo-vinyl]-, 3-[2-(4-chlorophenyl)-2-bromo-vinyl]-, 3-[2-(3,4-dichlorophenyl)-2-bromo-vinyl]-, 3-[2-(4-fluorophenyl)-2-bromo-vinyl]-, 3-[2-(4-tert.-butylphenyl)-2-bromo-vinyl]-2,2-dimethyl-cyclopropanecarboxylic acid and -2,2-dimethylcyclopropanecarboxylic acid chloride.

4. Insecticidal and/or acaricidal agents, characterised in that they contain at least one substituted bromostyrylcyclopropanecarboxylic acid ester of the formula (I).

5. Use of substituted bromostyryl-cyclopropanecarboxylic acid esters of the formula (I) for comba-ting insects and/or arachnidae.

6. Process for combating insects and/or arachnidae, characterised in that substituted bromostyryl-cyclopropanecarboxylic acid esters of the formula (I) are allowed to act on insects and/or arachnidae and/or their environment.

7. Process for the preparation of insecticidal and/or acaricidal agents, characterised in that substituted bromostyrylcyclopropanecarboxylic acid esters of the formula (I) are mixed with extenders and/or surface-active agents.

**Claims for the contracting state: IT**

1. Substituted bromostyryl-cyclopropanecarboxylic acid esters of the formula (I)

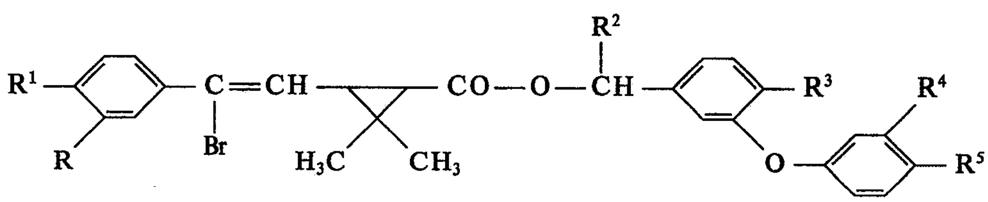

(I)

in which

R represents hydrogen, halogen, alkyl or alkoxy,
$R^1$ represents hydrogen, halogen, alkyl, alkoxy or alkylthio or, together with R, represents methylene-dioxy,
$R^2$ represents hydrogen, cyano or ethinyl and
$R^3$, $R^4$ and $R^5$ are identical or different and represent hydrogen or halogen, with the proviso that at least one of the radicals $R^3$, $R^4$ or $R^5$ represents halogen.

2. Process for the preparation of bromostyryl-cyclopropanecarboxylic acid phenoxybenzyl esters of the formula I according to Claim 1,

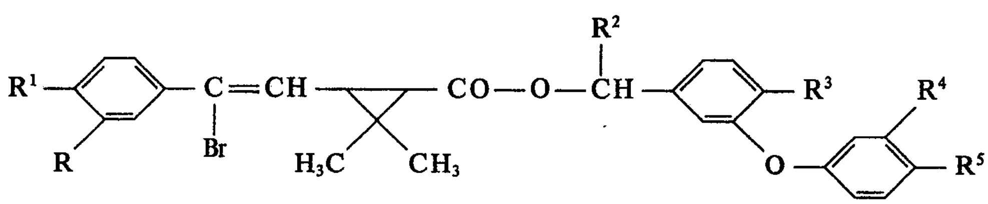

(I)

in which

R represents hydrogen, halogen, alkyl or alkoxy,
$R^1$ represents hydrogen, halogen, alkyl, alkoxy or alkylthio or, together with R, represents methylene-dioxy,
$R^2$ represents hydrogen, cyano or ethinyl and
$R^3$, $R^4$ and $R^5$ are identical or different and represent hydrogen or halogen, with the proviso that at least one of the radicals $R^3$, $R^4$ or $R^5$ represents halogen,

characterised in that bromostyryl-cyclopropanecarboxylic acids of the formula II

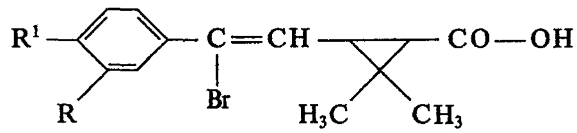

(II)

in which

R und $R^1$ have the meaning indicated above, or reactive derivatives thereof are reacted with substituted phenoxybenzyl alcohols of the formula III

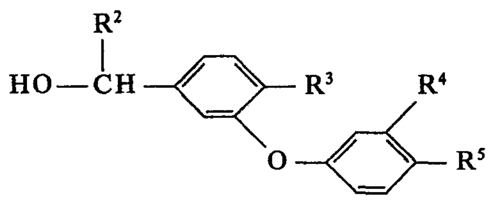

(III)

in which

$R^2$, $R^3$, $R^4$ and $R^5$ have the meaning indicated above, or with reactive derivatives thereof, if appropriate in the presence of an acid acceptor and if appropriate using one or more diluents.

3. Bromostyryl-cyclopropanecarboxylic acids of the formula II

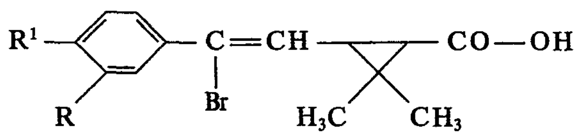

(II)

in which

R and $R^1$ have the meaning indicated in Claim 1.

4. Process for the preparation of bromostyryl-cyclopropanecarboxylic acids of the formula II, characterised in that $\alpha$-bromo-benzyl-phosphonic acid esters of the formula IV

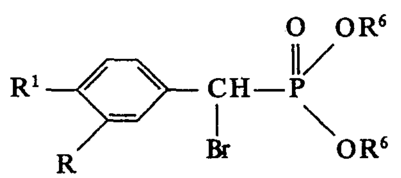

(IV)

in which

R and $R^1$ have the meaning indicated in Claim 1 and
$R^6$ represents alkyl or phenyl, or the two radicals $R^6$ together represent alkanediyl,

are reacted with formyl-cyclopropanecarboxylic acid esters of the formula V

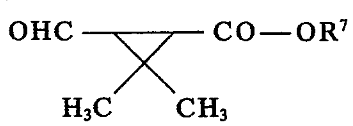

(V)

in which

$R^7$ represents alkyl,

in the presence of bases and if appropriate using diluents, and the bromostyryl-cyclopropanecarboxylic acid esters thereby formed are saponified by known methods, for example by heating with sodium hydroxide in aqueous alcohol, to give the corresponding bromostyryl-cyclopropanecarboxylic acids of the formula II.

5. Insecticidal and/or acaricidalagents, characterised in that they contain at least one substituted bromostyryl-cyclopropanecarboxylic acid ester of the formula (I).

6. Use of substituted bromostyryl-cyclopropanecarboxylic acid esters of the formula (I) for combating insects and/or arachnidae.

7. Process for combating insects and/or arachnidae, characterised in that substituted bromostyryl-cyclopropanecarboxylic acid esters of the formula (I) are allowed to act on insects and/or arachnidae and/or their environment.

8. Process for the preparation of insecticidal and/or acaricidal agents, characterised in that substituted bromostyryl-cyclopropanecarboxylic acid esters of the formula (I) are mixed with extenders and/or surface-active agents.

**Revendications pour les Stats Contractants: BE, CH, DE, FR, GB, NL.**

1. Esters substitués d'acides bromostyryl-cyclopropanecarboxyliques de formule (I)

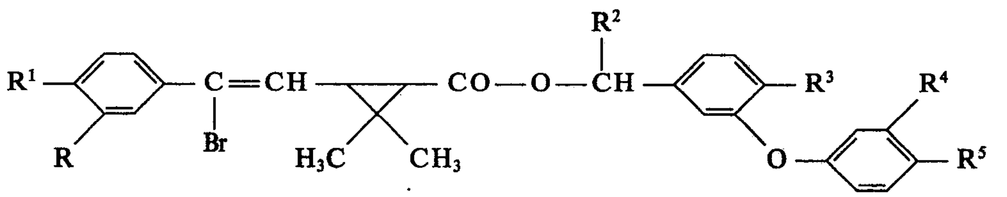

(I)

dans laquelle

R représente l'hydrogène, un halogène, un groupe alkyle ou un groupe alkoxy,
$R^1$ est l'hydrogène, un halogène, un groupe alkyle, alkoxy, alkylthio, ou forme conjointement avec R un groupe méthylènedioxy,
$R^2$ est l'hydrogène, le groupe cyano ou le groupe éthynyle,
$R^3$, $R^4$ et $R^5$ sont égaux ou différents et représentent l'hydrogène ou un halogène, sous réserve qu'au moins l'un des restes $R^3$, $R^4$ ou $R^5$ représente un halogène.

2. Procédé de production d'esters phénoxybenzyliques d'acides bromostyryl-cyclopropanecarboxyliques de formule I suivant la revendication 1

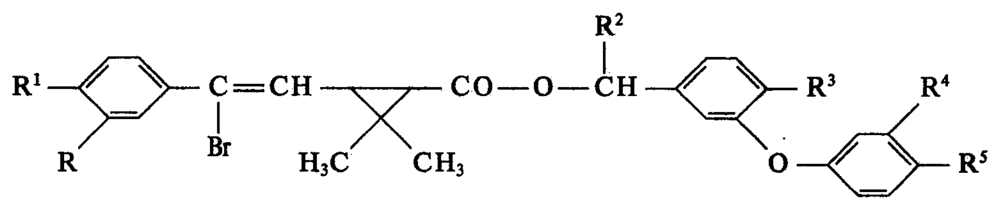

(I)

dans laquelle

R est l'hydrogène, un halogène, un groupe alkyle ou un groupe alkoxy,
$R^1$ est l'hydrogène, un halogène, un groupe alkyle alkoxy, alkylthio, ou forme conjointement avec R un groupe méthylènedioxy,
$R^2$ est l'hydrogène, le groupe cyano ou le groupe éthynyle,
$R^3$, $R^4$ et $R^5$ sont égaux ou différents et représentent l'hydrogène ou un halogène, sous réserve qu'au moins l'un des restes $R^3$, $R^4$ ou $R^5$ soit un halogène,

caractérise en ce qu'on fait réagir des acides bromostyryl-cyclopropanecarboxyliques de formule II

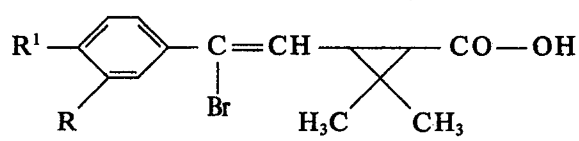

(II)

dans laquelle

R und $R^1$ ont la définition indiquée ci-dessus, ou des dérivés réactifs de ces acides, avec des alcools phénoxybenzyliques substitués de formule III

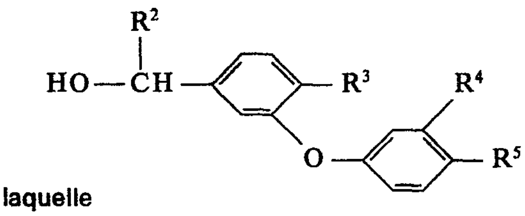

(III)

dans laquelle

$R^2$, $R^3$, $R^4$ et $R^5$ ont la définition indiquée ci-dessus, ou avec des dérivés réactifs de ces alcools, éventuellement en présence d'un accepteur d'acide et en utilisant le cas échéant un ou plusieurs diluants.

3. Acides bromostyryl-cyclopropanecarboxyliques de formule II

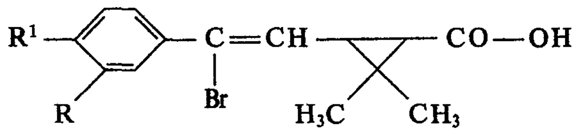

(II)

dans laquelle

R et $R^1$ ont la définition indiquée dans la revendication 1, à l'exception des composés acide

3-[2-phényl-2-bromovinyle]-, 3-[2-(4-chlorophényl)-2-bromovinyle]-, 3-[2-(3,4-dichlorophenyl)-2-bromovinyle]-, 3-[2-(4-fluorophényl)-2-bromovinyle]-, 3-[2-(4-tertiobutylphényl)-2-bromovinyl]-2,2-diméthylcyclopropanecarboxylique et du chlorure d'acide correspondant.

4. Compositions insecticides et/ou acaricides, caractérisées par une teneur en au moins un ester d'acide bromostyryl-cyclopropanecarboxylique substitué de formule (I).

5. Utilisation d'esters d'acides bromostyryl-cyclopropanecarboxyliques substitués de formule (I) pour combattre des insectes et/ou des acariens.

6. Procédé pour combattre des insectes et/ou des acariens, caractérisé en ce qu'on fait agir des esters d'acides bromostyryl-cyclopropanecarboxyliques substitués de formule (I) sur des insectes et/ou des acariens et/ou sur leur habitat.

7. Procédé de préparation de compositions insecticides et/ou acaricides, caractérisé en ce qu'on mélange des esters d'acides bromostyryl-cyclopropanecarboxyliques substitués de formule (I) avec des diluants et/ou des agents tensio-actifs.

**Revendications pour l' Etat Contractant: IT.**

1. Esters substitués d'acides bromostyryl-cyclopropanecarboxyliques de formule (I)

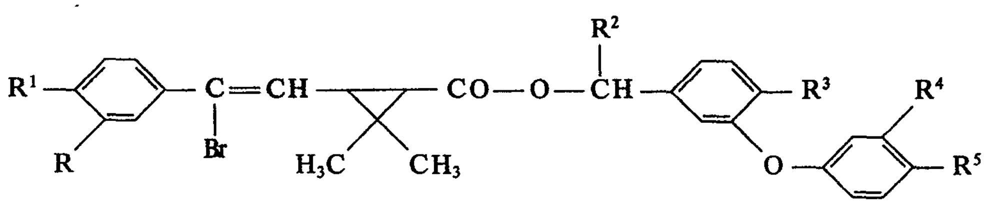

(I)

dans laquelle

R représente l'hydrogène, un halogène, un groupe alkyle ou un groupe alkoxy,
$R^1$ est l'hydrogène, un halogène, un groupe alkyle, alkoxy, alkylthio, ou forme conjointement avec R un groupe méthylènedioxy,
$R^2$ est l'hydrogène, le groupe cyano ou le groupe éthynyle,
$R^3$, $R^4$ et $R^5$ sont égaux ou différents et représentent l'hydrogène ou un halogène, sous réserve qu'au moins l'un des restes $R^3$, $R^4$ ou $R^5$ représente un halogène.

2. Procédé de production d'esters phénoxybenzyliques d'acides bromostyryl-cyclopropanecarboxyliques de formule I suivant la revendication 1

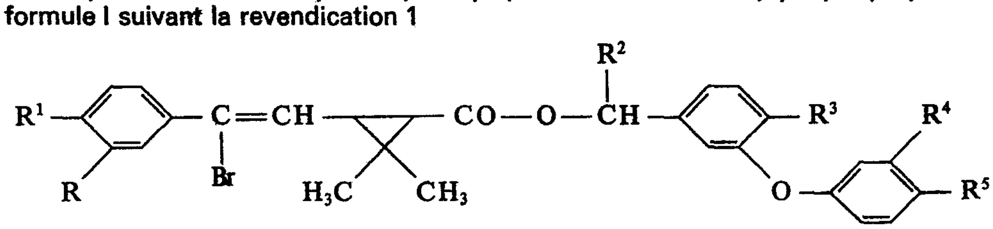

(I)

dans laquelle

R est l'hydrogène, un halogène, un groupe alkyle ou un groupe alkoxy,
$R^1$ est l'hydrogène, un halogène, un groupe alkyle alkoxy, alkylthio, ou forme conjointement avec R un groupe méthylènedioxy,
$R^2$ est l'hydrogène, le groupe cyano ou le groupe éthynyle,
$R^3$, $R^4$ et $R^5$ sont égaux ou différents et représentent l'hydrogène ou un halogène, sous réserve qu'au moins l'un des restes $R^3$, $R^4$ ou $R^5$ soit un halogène,

caractérise en ce qu'on fait réagir des acides bromostyryl-cyclopropanecarboxyliques de formule II

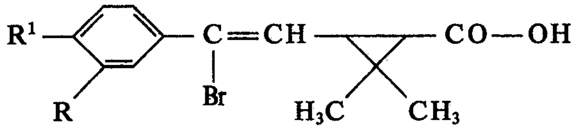

(II)

dans laquelle

R et $R^1$ ont la définition indiquée ci-dessus, ou des dérivés réactifs de ces acides, avec des alcools phénoxybenzyliques substitués de formule III

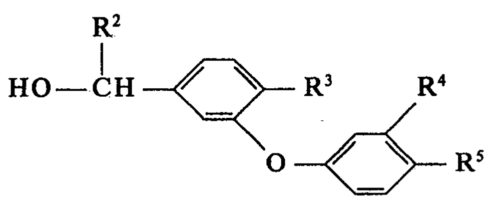

(III)

dans laquelle

$R^2$, $R^3$, $R^4$ et $R^5$ ont la définition indiquée ci-dessus, ou avec des dérivés réactifs de ces alcools, éventuellement en présence d'un accepteur d'acide et en utilisant le cas échéant un ou plusieurs diluants.

3. Acides bromostyryl-cyclopropanecarboxyliques de formule II

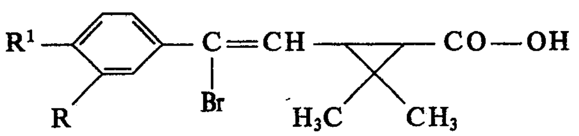

(II)

dans laquelle

R et $R^1$ ont la définition indiquée dans la revendicarion 1.

4. Procédé de production d'acides bromostyryl-cyclopropanecarboxyliques de formule II, caractérisé en ce qu'on fait réagir des esters d'acides $\alpha$-bromobenzylphosphoniques de formule IV

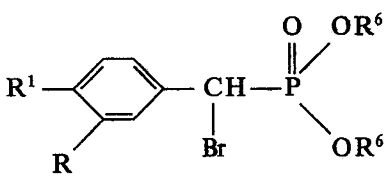

(IV)

dans laquelle

R et $R^1$ ont la définition indiquée dans la revendication 1 et
$R^6$ est un groupe alkyle ou phényle ou bien les deux restes $R^6$ forment ensemble un groupe alcanediyle,

avec des esters d'acides formyl-cyclopropanecarboxyliques de formule V

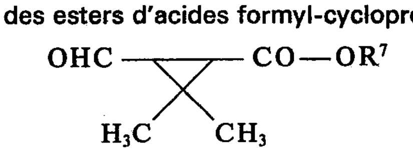

(V)

dans laquelle

$R^7$ est un groupe alkyle,

en présence de bases et le cas échéant en utilisant des diluants, et on saponifie les esters d'acides bromostyryl-cyclopropanecarboxyliques ainsi formés par des procédés connus, par exemple par chauffage avec de l'hydroxyde de sodium dans l'alcool aqueux pour former les acides bromostyryl-cyclopropanecarboxyliques correspondants de formule II.

5. Compositions insecticides et acaricides, caractérisées par une teneur en au moins un ester d'acide bromostyryl-cyclopropanecarboxylique substitué de formule (I).

6. Utilisation d'esters d'acides bromostyryl-cyclopropanecarboxyliques substitués de formule (I) pour combattre des insectes et/ou des acariens.

7. Procédé pour combattre des insectes et/ou des acariens, caractérisé en ce qu'on fait agir des esters d'acides bromostyryl-cyclopropanecarboxyliques substitués de formule (I) sur des insectes et/ou sur des acariens et/ou sur leur habitat.

8. Procédé de préparation de compositions insecticides et/ou acaricides, caractérisé en ce qu'on mélange des esters d'acides bromostyryl-cyclopropanecarboxyliques substitués de formule (I) avec des diluants et/ou des agents tensio-actifs.